(19) Europäisches Patentamt
European Patent Office
Office européen des brevets

(11) Veröffentlichungsnummer : **0 482 347 B1**

# (12) EUROPÄISCHE PATENTSCHRIFT

(45) Veröffentlichungstag der Patentschrift : **09.06.93 Patentblatt 93/23**

(51) Int. Cl.$^5$ : **C07C 251/88,** C07C 241/00, C07C 211/36

(21) Anmeldenummer : **91115713.9**

(22) Anmeldetag : **17.09.91**

## (54) Bis(3-cyano-3,5,5-trimethyl-cyclohexyliden)-azin, Verfahren zu seiner Herstellung und Weiterverarbeitung zu 3-(Aminomethyl)-3,5,5-trimethylcyclohexylamin.

(30) Priorität : **23.10.90 DE 4033609**
**14.06.91 DE 4119577**

(43) Veröffentlichungstag der Anmeldung : **29.04.92 Patentblatt 92/18**

(45) Bekanntmachung des Hinweises auf die Patenterteilung : **09.06.93 Patentblatt 93/23**

(84) Benannte Vertragsstaaten : **AT BE CH DE ES FR GB IT LI NL**

(56) Entgegenhaltungen :
**DE-A- 3 011 656**
**DE-A- 3 021 955**

(56) Entgegenhaltungen :
**DE-B- 1 229 078**
**DE-B- 2 312 374**
**GB-A- 2 027 026**
**PATENT ABSTRACTS OF JAPAN, unexamined applications, C Field, Band 11, Nr. 353, 18. November 1987 THE PATENT OFFICE JAPANESE GOVERNMENT Seite 5 C 457**

(73) Patentinhaber : **Degussa Aktiengesellschaft**
**Weissfrauenstrasse 9**
**W-6000 Frankfurt am Main 1 (DE)**

(72) Erfinder : **Huthmacher, Klaus, Dr.**
**Lärchenweg 18**
**W-6460 Gelnhausen (DE)**
Erfinder : **Schmitt, Hermann**
**Berliner Strasse 24**
**W-6451 Rodenbach (DE)**

EP 0 482 347 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung richtet sich auf die neue Verbindung Bis(3-cyano-3,5,5-trimethylcyclohexyliden)-azin, ein Verfahren zu seiner Herstellung aus 1,3,3-Trimetyhl-5-oxo-cyclohexan-carbonitril sowie seiner Weiterverarbeitung zu 3-(Aminomethyl)-3,5,5-trimethylcyclohexylamin.

3-(Aminomethyl)-3,5,5-trimethylcyclohexylamin, nachfolgend als Isophorondiamin bezeichnet, wird als Ausgangsprodukt zur Herstellung von Isophorondiisocyanat, als Aminkomponente für Polyamide und als Härter für Epoxidharze verwendet.

Bisher wird Isophorondiamin (IPDA) durch reduktive Aminierung von 1,3,3-Trimethyl-5-oxo-cyclohexancarbonitril, nachfolgend als Isophoronnitril (IPN) bezeichnet, in Gegenwart von Ammoniak und üblichen Hydrierkatalysatoren gewonnen. Das als Ausgangsverbindungen dienende Isophoronnitril ist durch Anlagerung von Cyanwasserstoff an Isophoron erhältlich - vgl. DE-Patentanmeldung P 39 42 371.9.

Nach dem Verfahren der DE-PS 12 29 078 werden Ammoniak und IPN im Molverhältnis von 10-30 zu 1 eingesetzt, um IPDA zu gewinnen. Außer dem gewünschten IPDA entstehen aber in größerer Menge Nebenprodukte, wie insbesondere 3-(Aminomethyl)-3,5,5-trimethylcyclohexanol (=Isophoronaminoalkohol (IPAA)), 1,3,3-Trimethyl-6-aza-bicyclo- 3,2,1 -octan und Dihydroisophorylamin. Beispielhaft wird eine Ausbeute von bis zu 81,4 % IPDA angegeben, jedoch fehlen weitere Reinheitsangaben. Die genannte Ausbeute erwies sich, wie von verschiedenen Seiten festgestellt wurde, als nicht reproduzierbar.

Im Bestreben, die Ausbeute an IPDA zu erhöhen und den Zwangsanfall an IPAA zu minimieren, wurde gemäß DE-OS 30 11 656 das aus der DE-PS 12 29 078 bekannte Verfahren dahingehend abgeändert, daß in einer ersten Stufe IPN katalysatorfrei mit überschüssigem Ammoniak in 1,3,3-Trimethyl-5-imino-cyclohexancarbonitril überführt und dieses in einer zweiten Stufe zu IPDA hydriert wurde. In der 2. Stufe mußte ein erheblicher Überschuß an Ammoniak eingesetzt werden. Diese Arbeitsweise macht eine aufwendige Druckdestillation zwecks Rückgewinnung und Rezyklierung des Ammoniaks erforderlich. Beispielsgemäß wurde im Verfahren der DE-OS 30 11 656 trotz eines Mengenverhältnisses von etwa 5 kg Ammoniak pro 1 kg IPN nur eine Reaktionsausbeute von 83,7 % erzielt; über die Ausbeute an isoliertem IPDA und dessen Reinheit werden keine Angaben gemacht.

Daß ein weiterer Bedarf bestand, die Verfahren der vorgenannten Dokumente zu verbessern, folgt aus der DE-OS 30 21 955. Gemäß Vergleichsbeispiel 1 der DE-OS 30 21 955 gelangt man trotz eines IPN/$NH_3$-Volumenverhältnisses von 1 zu 10 im Verfahren analog DE-PS 12 29 078 nur zu einer IPDA-Ausbeute von 48 %. Gemäß den Vergleichsbeispielen 2 und 3 der DE-OS 30 21 955, durchgeführt analog DE-OS 30 11 656, konnte eine Reaktionsausbeute von ca. 70 % bzw. 90 % erreicht werden; die hohe Ausbeute erforderte aber eine lange Reaktionszeit für die erste Stufe und ein IPN/$NH_3$-Volumenverhältnis von 1 zu 10 in der zweiten Stufe. Zu dem Nachteil des hohen Ammoniaküberschusses kommt also noch eine wirtschaftlich bedeutsame Minderung der Raum-Zeit-Ausbeute.

Die lange Reaktionszeit für die erste Stufe - Iminbildung - im Verfahren der DE-OS 30 11 656 konnte gemäß DE-OS 30 21 955 dadurch reduziert werden, daß ein Iminbildungskatalysator eingesetzt wurde. Die Hydrierung in der zweiten Stufe erforderte aber weiterhin ein Volumenverhältnis von Isophoronnitril zu Ammoniak von 1 zu 10 bis 20 und damit eine teure Anlage zur Druckdestillation; als weiterer Nachteil kommt eine aufwendige Reaktionsführung hinzu.

Das Verfahren der JP-A 62-123154 richtet sich auf die reduktive Aminierung von IPN zur Herstellung von IPDA, wobei versucht wurde, den erforderlichen Ammoniaküberschuß zu vermindern und auf die Vorreduktion des trägergebundenen Katalysators zu verzichten. Bei Verwendung der 1- bis 20-fachen, vorzugsweise 5- bis 10-fachen Molmenge Ammoniak, bezogen auf IPN, sowie Raney-Kobalt als Katalysator, einem Druck von 50 bis 150 bar und einer Temperatur von 50 bis 150°C soll es nach dem genannten Verfahren möglich sein, IPDA in hoher Ausbeute zu gewinnen - der IPDA-Anteil in den beispielsgemäßen Reaktionsgemischen betrug etwa 83-89 %, der IPAA-Anteil 4-6 % (GC-Flächen-%). In Anbetracht des hohen Anteils an schwierig abtrennbarem IPAA muß bei der destillativen Aufarbeitung mit einem nicht unbeträchtlichen Ausbeuteverlust an IPDA gerechnet werden. Bei der Nacharbeitung des Verfahrens der JP-A 62 123154 von der Anmelderin des vorliegenden Dokuments konnten die Aussagen der JP-A 62 123154 in keiner Weise bestätigt werden. Wie aus den Vergleichsbeispielen 1 und 2 hervorgeht, wird IPN unter den angegebenen Bedingungen nur unzureichend zu IPDA hydriert.

Aus dem zuvor gewürdigten Stand der Technik wird deutlich, daß mittels reduktiver Aminierung von Isophoronnitril mit Ammoniak und Wasserstoff über das Zwischenprodukt 1,3,3-Trimethyl-5-imino-cyclohexancarbonitril das gewünschte Isophorondiamin (IPDA) nur dann in guter Ausbeute und mit begrenztem Zwangsanfall an Isophoronaminoalkohol (IPAA) erhalten werden konnte, wenn Ammoniak in sehr großem Überschuß zum Einsatz kam. Dieser Überschuß an Ammoniak machte eine aufwendige Anlage mit Einrichtungen zur Druckdestillation und Rückgewinnung des Iminbildungskatalysators erforderlich.

Aufgabe der vorliegenden Erfindung ist, einen neuen Weg zur Herstellung von Isophorondiamin aus Isophoronnitril aufzuzeigen, der es gestattet, die Nachteile der vorbekannten Verfahren zu mindern und insbesondere den Einsatz an Ammoniak so zu reduzieren, daß auf eine Druckdestillation verzichtet werden kann; gleichzeitig sollten IPDA in hoher Ausbeute und praktisch kein IPAA gebildet werden.

Gefunden wurde, daß bei der Umsetzung von Isophoronnitril mit einer Quelle für Hydrazin im Molverhältnis von im wesentlichen 2:1 ein bisher nicht bekanntes Azin, nämlich Bis(3-cyano-3,5,5-trimethylcyclohexyliden)-azin, nachfolgend IPN-azin genannt, in praktisch quantitativer Ausbeute entsteht. Das IPN-azin kann seinerseits in Gegenwart von Ammoniak und Katalysatoren zu Isophorondiamin hydriert werden (siehe Reaktionsschema).

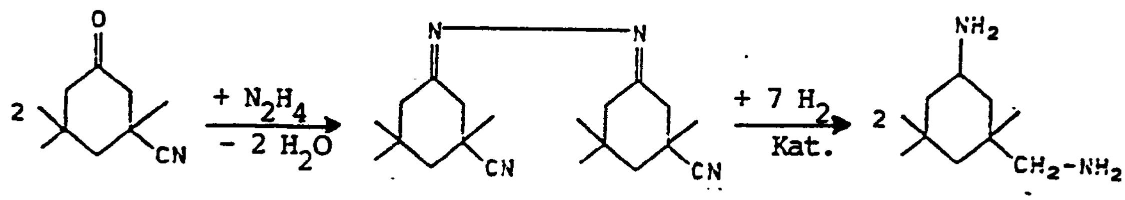


(IPN) (IPN-azin) (IPDA)

Das als Zwischenprodukt auftretende IPN-azin, das mittels üblicher Methoden aus dem Reaktionsgemisch seiner Herstellung isoliert werden kann, ist neu, und seine Struktur wurde mittels analytischer und spektroskopischer Methoden nachgewiesen. Es konnte nicht erwartet werden, daß bei der Weiterverarbeitung des IPN-azins sehr reines Isophorondiamin ohne Zwangsanfall von Nebenprodukten erhalten werden kann.

Das Verfahren zur Herstellung des IPN-azins ist dadurch gekennzeichnet, daß man 1,3,3-Trimethyl-5-oxo-cyclohexancarbonitril (=IPN) und einer Quelle für Hydrazin im Molverhältnis von im wesentlichen 2 zu 1 in Gegenwart eines Lösungsmittels umsetzt und, soweit erwünscht, des IPN-azin mittels üblicher Methoden aus dem Reaktionsgemisch isoliert.

Als Quelle für Hydrazin kommen Hydrazin, Hydrazinhydrat und wäßrige Lösungen derselben in unterschiedlichen Konzentrationen und Hydrazinsalze infrage, wobei Hydrazinhydrat und wäßrige Lösungen derselben besonders bevorzugt werden. Im Falle der Verwendung von Hydrazinsalzen ist eine anschließende Neutralisation des Salzbildners erforderlich. Wegen des Salzanfalls werden deshalb Hydrazinsalze gegenüber Hydrazin und Hydrazinhydrat weniger bevorzugt.

Gemäß der Formel des IPN-azins sind pro Mol Quelle für Hydrazin zwei Mol Isophoronnitril erforderlich. Unter dem Molverhältnis "im wesentlichen 2:1" wird ein solches verstanden, das zwar vorzugsweise 2:1 beträgt, einer der Reaktionspartner aber in bis zu 10 %igem Überschuß eingesetzt werden kann.

Die Umsetzung besteht darin, daß die Edukte in Gegenwart eines Lösungsmittels zusammengebracht werden, wobei das Reaktionsgemisch zweckmäßigerweise gerührt wird. Es können beide Edukte parallel dem Reaktionsgefäß zugeführt werden, wobei sich das Lösungsmittel in diesem befindet oder mit mindestens einem der Edukte eingebracht wird. Selbstverständlich kann auch einer der Reaktionspartner in dem Lösungsmittel vorgelegt und durch Zudosieren des zweiten Reaktionspartners zur Reaktion gebracht werden. Die Umsetzung kann diskontinuierlich oder kontinuierlich durchgeführt werden.

Die Reaktion verläuft exotherm. Die Reaktionstemperatur ist unkritisch, vorzugsweise liegt sie im Bereich von 20 °C bis 120 °C; besonders bevorzugt erfolgt die Umsetzung bei Siedetemperatur des verwendeten Lösungsmittels.

Als Lösungsmittel kommen gegen IPN und Hydrazin inerte, sowohl wäßrige als auch organische Lösungsmittel infrage; letztere werden bevorzugt. Das Lösungsmittel dient als flüssiges Reaktionsmedium. Mindestens eines der Edukte soll in dem Lösungsmittel zumindest teilweise, vorzugsweise ganz löslich sein; vorzugsweise sind beide Edukte zumindest teilweise und zweckmäßigerweise vollständig in dem Lösungsmittel löslich - diese Eigenschaft läßt sich durch einen Vorversuch leicht ermitteln. Es ist besonders günstig, solche Lösungsmittel für die Umsetzung zu wählen, in welchen das entstehende IPN-azin nur wenig löslich ist und somit während der Umsetzung zumindest teilweise als Feststoff ausfällt - hierdurch wird die Gleichgewichtseinstellung der Azinbildung gefördert. Lösungsmittelgemische, auch wäßrig-organische, sind einsetzbar, bringen aber keine Vorteile. Lösungsmittel mit einem Siedepunkt unter 120 °C werden bevorzugt. Besonders geeignet sind niedere ein- oder zweiwertige Alkohole, insbesondere einwertige $C_1$- bis $C_4$-Alkohole und aliphatische oder cyclische Ether mit bis zu 6 C-Atomen; in Betracht kommen auch aliphatische oder cycloaliphatische Kohlenwasserstoffe, wie z. B. Cyclohexan. Besonders zweckmäßig ist es für die Azinherstellung, jenes Lösungsmittel einzusetzen, das auch bei der Weiterverarbeitung zu IPDA zum Einsatz kommen soll.

Das IPN-azin kann durch Abdestillieren des Lösungsmittels oder, sofern das IPN-azin in dem Lösungsmittel kaum löslich ist, durch Fest-Flüssig-Phasentrennung aus dem Reaktionsgemisch isoliert werden. Sofern erforderlich, kann sich an die Isolierung eine Reinigung anschließen, etwa durch Nachwaschen oder Umkristallisieren. Da die Umsetzung überraschenderweise praktisch quantitativ erfolgt und kaum Nebenprodukte gebildet werden, erübrigen sich im allgemeinen eine Isolierung und Reinigung vor der Weiterverarbeitung zu IPDA.

Die Weiterverarbeitung des IPN-azins zu Isophorondiamin (IPDA) ist dadurch gekennzeichnet, daß man das IPN-azin in Gegenwart eines organischen Lösungsmittels, Ammoniak und eines Katalysatorsystems aus der Reihe (a) eines kobalt- oder nickelhaltigen Raney-Katalysators und eines Cokatalysators aus der Reihe von Salzen der Elemente Aluminium, Kobalt, Nickel, Lanthan, Yttrium, Cer, Ru, Rh, Pd, Ir und Pt oder von trägergebundenen Edelmetallen aus der Reihe Ru, Rh, Pd, Ir, Pt oder (b) eines trägergebundenen Ruthenium-, Palladium- oder Platinkatalysators bei einem Druck von 3 bis 30 MPa und einer Temperatur von 50 bis 150 °C mit Wasserstoff hydriert und das Reaktionsgemisch nach Abtrennung der Feststoffe destillativ aufarbeitet.

Die Hydrierung des IPN-azins erfolgt mit Wasserstoff, wobei gleichzeitig die Nitrilgruppen hydriert und die Azingruppe hydrierend gespalten werden. Der bevorzugte Druckbereich beträgt 3 bis 15 MPa, die bevorzugte Temperatur 80 bis 120 °C. Sowohl die Gegenwart von Ammoniak als auch die Verwendung der Katalysatorsysteme (a) oder (b) sind wesentliche Verfahrensmerkmale. Der Cokatalysator des Katalysatorsystems (a) steigert einerseits die Hydrieraktivität der Raney-Katalysatoren, andererseits ermöglicht er die Azinspaltung.

Die Hydrierung des IPN-azins zu IPDA kann diskontinuierlich als auch kontinuierlich erfolgen, wobei übliche Hydrierreaktoren, wie sie für Hydrierungen unter Verwendung von Suspensionskatalysatoren Einsatz finden, verwendbar sind; beispielhaft seien Rührautoklaven und Schlaufenreaktoren genannt.

Als Lösungsmittel kommen solche in Betracht, welche eine ausreichende Lösekraft für IPN-azin und IPDA bei der gewählten Hydriertemperatur aufweisen. Nach der Hydrierung soll IPDA vollständig in Lösung sein, um Feststoffe, also den Katalysator und gegebenenfalls Cokatalysator durch einfache Filtration vom Reaktionsgemisch abtrennen zu können. Geeignet sind beispielsweise niedere Alkohole, insbesondere einwertige $C_1$- bis $C_4$-Alkohole, aliphatische und cycloaliphatische Mono-und Diether, insbesondere solche mit bis zu 6 C-Atomen, aber auch allgemein aliphatische und cycloaliphatische Kohlenwasserstoffe, wie Cyclohexan, in Betracht. Vorzugsweise werden solche Lösungsmittel ausgewählt, welche sich leicht vom Reaktionsgemisch abdestillieren lassen, also einen Siedepunkt unter 120 °C aufweisen. Das Lösungsmittel muß unter den Hydrierbedingungen stabil sein.

Wie schon ausgeführt, ist die Anwesenheit von Ammoniak wesentlich. Besonders bewährt haben sich Mengen von 50 bis 500 g $NH_3$ pro kg IPN-azin, es sind aber auch kleinere und größere Mengen einsetzbar. Eine Steigerung der $NH_3$-Menge auf über 2 kg pro kg IPN-azin ist aus wirtschaftlichen Erwägungen wenig sinnvoll, weil sich die Raum-Zeit-Ausbeute und Reinheit des IPDA's nicht weiter verbessern. Durch eine $NH_3$-Menge unter 50 g pro kg IPN-azin sinken die Ausbeute an IPDA und Reinheit des IPDA-Rohproduktes.

Im Prinzip kann anstelle Ammoniak auch Hydrazin verwendet werden, jedoch wird diese Alternative als weniger zweckmäßig angesehen.

Als Katalysatoren des Katalysatorsystems (a) finden die aus NiAl- und CoAl-Legierungen, welche auch zusätzlich weitere Metalle, wie z. B. Mangan, aufweisen können, in allgemein bekannter Weise herstellbaren und auch im Handel erhältlichen Raney-Katalysatoren Anwendung.

Im Katalysatorsystem (a) erfüllen zwei Arten von Cokatalysatoren, nämlich Salze der Elemente Al, Co, Ni, Y, Lanthaniden Ru, Rh, Pd, Ir, Pt und trägergebundene Edelmetalle der Reihe Ru, Rh, Pd, Ir, Pt die an sie gestellte Funktion. Unter den Salzen, welche in Form einer Lösung oder als Pulver dem Reaktionsansatz zugefügt oder vorab mit dem Katalysator zusammengebracht werden, eignen sich Salze von Mineralsäuren oder organischen Säuren; bevorzugt werden Halogenide und Azetate in wasserfreier oder wasserhaltiger Form. Besonders vorteilhaft sind Chloride von Al, Co, Ni, Y, La, Ce, insbesondere von Co und Ni. üblicherweise wird das Cokatalysator-Salz in einer Menge von 0,01 bis 0,5 Mol, insbesondere 0,05 bis 0,2 Mol pro Mol Raney-Nickel bzw. Raney-Cobalt eingesetzt. Bei den trägergebundenen Edelmetallcokatalysatoren handelt es sich um solche auf feinverteilten Trägern, wie z. B. auf Aktivkohle, Kieselsäure, Aluminiumoxid oder silikatischen Stoffen, welche in an sich bekannter Weise herstellbar oder käuflich erhältlich sind.

Ein Vorteil des Katalysatorsystems (b) besteht darin, daß dieses ohne Cokatalysator verwendbar ist. Trägergebundene Ruthenium-Katalysatoren sind gegenüber den Pd- und Pt-Katalysatoren bevorzugt, weil mit ihnen höhere Ausbeuten und Produktreinheiten erzielbar sind. Als Trägermaterial kommen die dem Fachmann bekannten feinteiligen Stoffe infrage, wie beispielsweise Kohle, Aluminiumoxid, Silikate sowie Oxide von Ti, Zr, Sn, La, Ce, wobei Kohle und Aluminiumoxid besonders bevorzugt sind. Die trägergebundenen Edelmetallkatalysatoren lassen sich in an sich bekannter Weise herstellen oder sind teilweise auch käuflich erhältlich.

Es war bekannt (JP 01203355 Az (1989) = Chem. Abstr. 112 (9): 76465 g), Azine aromatischer Aldehyde bei der Hydrierung mit Wasserstoff in essigsaurer Lösung unter Verwendung von Rh auf Aktivkohle als Katalysator hydrierend zu spalten Dieses Verfahren führt aber bei der gewünschten Hydrierung des IPN-azins zu

IPDA nicht zum Erfolg. Es war daher überraschend, daß trägergebundene Ru-, Pd- und Pt-Katalysatoren im erfindungsgemäßen Verfahren die gewünschte gleichzeitige Hydrierung und Azinspaltung ermöglichen. Eine Azinspaltung mit dem System Raney-Katalysator / Salz-Cokatalysator ist bisher nie beschrieben oder nahegelegt worden.

Durch die erfindungsgemäße Hydrierung des IPN-azins, das aus IPN, wie zuvor dargelegt, leicht erhältlich ist und vorteilhafterweise in Form einer Suspension aus seiner Herstellung eingesetzt wird, gelingt es, Isophorondiamin in einfacher Weise in hoher Ausbeute zu gewinnen. Der bisher erforderliche hohe $NH_3$-Überschuß und damit eine Druckdestillation sind nicht erforderlich. Das IPDA-Rohprodukt, erhalten nach der Hydrierung, Abtrennung des Katalysators und Cokatalysators und Abdestillieren des Ammoniaks und des Lösungsmittels, ist frei von dem als störend empfundenen Isophoronaminoalkohol. Die weitere Reinigung des IPDA-Rohprodukts ist mittels üblicher destillativer Methoden leicht möglich.

**Beispiel 1**

Herstellung von IPN-azin aus IPN:

495,4 g (3 Mol) Isophoronnitril werden zu 1200 ml Methanol Raumtemperatur (=RT) beigegeben. Unter Rühren werden 93,9 g (1,5 Mol) Hydrasurhydrat (80 %ig) zudosiert; die Reaktionslösung wird zum Sieden erhitzt. Unter Rühren läßt man Abkühlen, wobei das IPN-azin ausfällt. Nach Filtration und Nachwaschen mit kaltem Methanol wird im Vakuum bei 60 °C getrocknet. IPN-azin 454 g, entsprechend 92,7 % d. Th. Schmelzpunkt 191 bis 194 °C, Elementaranalyse.

| | | | | | |
|---|---|---|---|---|---|
| C | ber.: 73,58 | H | ber.: 9,28 | N | ber.: 17,15 |
| | gef.: 73,40 | | gef.: 9,35 | | gef.: 17,10 |

IR-Spektrum: $\nu_{C=N}$ 2230 $cm^{-1}$; $\nu_{C=N}$ 1680 $CM^{-1}$

Das IPN-azin wurde außer mittels IR-Spektrum - siehe Figur 1/1 - mittels $^1$H- und $^{13}$C-NMR-Spektren charakterisiert.

**Beispiel 2**

Herstellung von IPN-azin aus Isophoron, HCN und $N_2H_4 \cdot H_2O$ ohne Isolierung des IPN:

Apparatur: 2 l Dreihalskolben, Rückflußkühler, Thermometer, Tropftrichter, Rührer.

752 ml (5 Mol) Isophoron und 3 g LiOH werden vorgelegt und unter Rühren auf 130 °C erhitzt. Innerhalb von 15 Minuten läßt man 118 ml (3 Mol) Cyanwasserstoff zutropfen, wobei die Temperatur auf 150 bis 155 °C ansteigt. Nach kurzem Nachrühren bei RT wird mit HCl auf einen pH-Wert 2-3 eingestellt. Anschließend wird bei vermindertem Druck und einer Innentemperatur bis 145 °C überschüssiges Isophoron abdestilliert - 254,8 g, $Kp_{14}$ 59-97 °C. Zum im wesentlichen IPN enthaltenden Destillationssumpf werden 1,2 l Methanol gegeben und dann die Li-Salze abfiltriert und mit 100 ml Methanol nachgewaschen. Zu den vereinigten Methanolphasen werden 75 ml (1,5 Mol) Hydrazinhydrat (100 %ig) in 20 Minuten zugetropft, wobei die Temperatur auf etwa 35 °C ansteigt und unter Rühren und Abkühlen die Kristallisation des IPN-azins beginnt. Nach 3 Stunden wird bei 20 °C abfiltiert, nachgewaschen und getrocknet. IPN-Azin 415,4 g = 84,8 % d. Th.; Schmelzpunkt 192-194 °C, Elementaranalyse.

| | | | | | |
|---|---|---|---|---|---|
| C | ber.: 73,58 | H | ber.: 9,26 | N | ber.: 17,15 |
| | gef.: 73,10 | | gef.: 9,86 | | gef.: 17,01 |

Aus dem Filtrat lassen sich weitere 30 g = 6,1 % d. Th. IPN-azin gewinnen.

**Beispiel 3**

163,2 g (0,50 Mol) IPN-azin werden in einem 2 l-Autoklaven mit Begasungsrührer in 850 ml Methanol und 150 ml wasserfreiem flüssigen Ammoniak aufgelöst. Nach Zugabe von 12,5 g Raney-Nickel und 6 g Kobaltchloridhexahydrat als Cokatalysator wird unter Rühren Wasserstoff bis 100 bar aufgepreßt und auf 110 °C erhitzt.

Nach beendeter Hydrierung wird der Katalysator abfiltriert; anschließend werden Ammoniak und das Lösungsmittel abgezogen, und der Rückstand im Vakuum über eine Kolonne destilliert.

Hauptlauf: 155,2 g (91,2 % d. Th.) IPDA
$Kp_{0,3}$: 74 - 76 °C
Rückstand: 3,2 g

Der Hauptlauf weist eine gaschromatiographisch bestimmte Reinheit von 99,2 % auf und enthält kein IPAA.

(GC-Bedingungen: Kapillarsäule DB5; Länge 30 m;
Einspritzblocktemp.: 250 °C;
Detektortemp.: 250 °C;
Temperaturprogramm 70-270 °C.)

**Beispiel 4**

Versuch wie in Beispiel 3, jedoch mit 1,5 Mol = 489,6 g IPN-azin, 2,65 l Methanol und 0,35 l wasserfreiem Ammoniak; als Katalysator wurden 35,0 g Raney-Kobalt und 5,0 g Kobaltchloridhexahydrat eingesetzt. Ausbeute 476,8 g (93,5 % d. Th.) IPDA.

**Beispiel 5**

Versuch gemäß Beispiel 4, jedoch mit 200 ml Ammoniak und 2,8 l Methanol. Ausbeute an IPDA: 454,4 g (89 % d. Th.); Reinheit 99,0 %, frei von IPAA.

**Beispiel 6**

Vesuch gemäß Beispiel 4, jedoch mit 50 ml Ammoniak und 2,9 l Methanol. Ausbeute an IPDA 410 g (80,3 % d. Th.); Reinheit 98,3 %.

**Beispiel 7**

Versuch gemäß Beispiel 4, jedoch mit 2,8 l Methanol und 75 ml Hydrazinhydrat (100 %ig) anstelle von Ammoniak. Ausbeute 357 g (70 % d. Th.) IPDA; Produktreinheit 98,3 %.

**Beispiel 8** (nicht erfindungsgemäß)

Beispiel 5 wurde wiederholt, wobei aber der Cokatalysator Kobaltchloridhexahydrat weggelassen wurde. Ausbeute an IPDA 352 g (69 % d. Th.) mit einer Reinheit von 98,2 %. Im Rückstand befanden sich wesentliche Anteile an Bis(3-aminomethyl-3,5,5-trimethyl-cyclohexyliden)-azin.

**Beispiel 9**

495,4 g (3 Mol) Isophoronnitril werden in 1,3 l Methanol bei 10 °C aufsuspendiert, danach werden langsam 75 ml (1,5 Mol) Hydrazinhydrat (100 %ig) zugegeben; dabei steigt die Temperatur auf 34 °C an. Bis zur vollständigen Ausfällung des IPN-azins wird noch nachgerührt. Die so erhaltene Suspension wird in einen 5 l-Rührautoklaven überführt, mit 1250 ml Methanol und 0,45 l wasserfreien Ammoniak versetzt und in Gegenwart von 35 g Raney-Nickel und 12,5 g Nickelchloridhexahydrat bei 110 °C und 100 bar Wasserstoffdruck hydriert. Nach dem Abkühlen wird der Katalysator abfiltiert, das Lösungsmittel abgezogen und der Rückstand im Vakuum destilliert.

Ausbeute: 475 g (93 % d. Th.) IPDA-Gehalt 99,3 %
Rückstand: 24,4 g

**Beispiel 10**

Herstellung von IPDA aus Isophoron über IPN (gemäß DE-Anmeldung P 39 42 371.9) und IPN-Azin - "Eintopf-Variante"

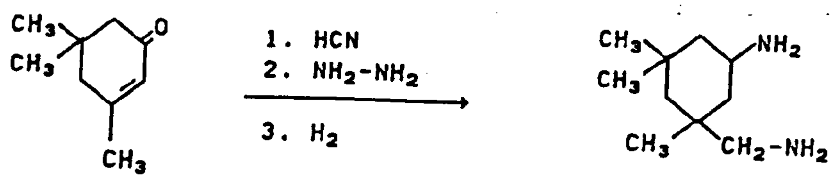


Einsatzstoffe:

| | |
|---|---|
| 752 ml | Isophoron (= 5 Mol) (IP) |
| 3 g | LiOH |
| 118 ml | Blausäure (= 3 Mol) |
| | Konz. HCl |
| 1,2 l | Methanol |
| 75 ml | Hydrazinhydrat (= 1,5 Mol) |
| 1 l | $NH_6$, flüssig |
| 100 g | Raney-Kobalt |
| 50 g | $CoCl_2 \cdot 6H_4O$ |

Die Umsetzung des Isophorons mit HCN erfolgt gemäß Beispiel 2. Nach Abdestillieren des Isophoronüberschusses - 270 g, $Kp_{14}$ 54-95 °C Innentemperatur 145 °C - wird der Destillationssumpf wie in Beispiel 2 weiterbehandelt und zu IPN-azin umgesetzt. Die IPN-azin-Suspension wurde in einen 5 l-Rührautoklaven überführt, mit dem Katalysator, Cokatalysator und 1,35 l Methanol versetzt; dann wurden 0,45 l Ammoniak (flüssig) und dann Wasserstoff aufgedrückt. Bei 110 °C und Aufrechterhaltung eines Drucks von 10 MPa wurde bis zum Ende der Wasserstoffaufnahme hydriert. Nach Ende der $H_2$-Aufnahme wurde entspannt, der Feststoff von der Lösung abfiltriert, der Rückstand mit 200 ml Methanol gewaschen. Die Lösung wurde im Vakuum von Methanol befreit, der Sumpf im Hochvakuum über eine 30 cm Vigreux-Kolonne destilliert:

| | | |
|---|---|---|
| Vorlauf: | 1,1 g | $Kp_{0,1}$: bis 75 °C |
| Hauptlauf: | 460 g = 90 % d.Th. | $Kp_{0,3}$: 75-78 °C |
| Rückstand: | 28,9 g | Innentemp. bis 140 °C |

Der Hauptlauf bestand aus Isophorondiamin (IPDA) in einer Reinheit (GC) von 99,1 %.

**Beispiele 11 bis 13**

Gemäß Beispiel 3, jedoch unter Verwendung der in der Tabelle angegebenen Cokatalysatoren, wurde IPN-azin zu IPDA hydriert. Die Cokatalysatoren, deren Menge sowie die Ausbeute an IPDA und Reinheit folgen aus der Tabelle.

Tabelle

| Beispiel-Nr. | 11 | 12 | 13 |
|---|---|---|---|
| Cokatalysator | $CeCl_3 \cdot 7H_2O$ | $AlCl_3$ | $PdCl_2$ |
| Menge Cokat.(g) | 6,5 | 2,4 | 3,8 |
| Ausbeute IPDA (% d. Th.) | 89,3 | 88,1 | 89,6 |
| Reinheit IPDA (%) | 98,8 | 98,5 | 98,6 |

**Beispiel 14**

Gemäß Beispiel 3, jedoch unter Verwendung eines trägergebundenen Edelmetalls - 40 g 5 % Rh auf Kohle mit 52 % Wasser - als Cokatalysator, wurde IPN-azin hydriert. Erhalten wurden bei $Kp_{0,3}$ 75-78 °C 150 g IPDA (=88,1 % d. Th.); Reinheit 99,1 %.

**Beispiel 15**

Die Hydrierung des IPN-azins erfolgte gemäß Beispiel 3, jedoch wurde kein cokatalytisch wirksames Salz zugesetzt, sondern ein solches durch Zugabe von 1 ml Essigsäure in situ aus einem Teil des Raney-Katalysators gebildet. Erhalten wurde IPDA in einer Ausbeute von 88,7 % d. Th. mit einer Reinheit von 99,3 %.

**Beispiel 16**

Die Hydrierung erfolgte gemäß Beispiel 3, wobei jedoch als Lösungsmittel Ethanol anstelle Methanol verwendet wurde: IPDA-Destillation 153,8 g = 90,4 % d. Th; IPDA-Gehalt: 99,4 %.

**Beispiel 17**

In einem Kreislaufreaktor werden in 33 kg Methanol und 5 kg Ammoniak 8,1 kg (24,85 Mol) IPN-azin vorgelegt und bei einem Wasserstoffdruck von 7 MPa auf 110 °C aufgeheizt. Nach Zugabe von 0,59 kg Raney-Ni und 0,207 kg Nickelchlorid-Hexahydrat als Cokatalysator wird bis zur Beendigung der Wasserstoffaufnahme (2,5 Stunden) hydriert. Nach dem Abkühlen wird der Katalysator abfiltriert und das Lösungsmittel mit dem Ammoniak abgezogen; der Rückstand wird im Vakuum fraktioniert.

Hauptlauf: 7,95 kg (94,1 % d. Th.) IPDA
$K_{P0,3}$: 75-77 °C; Reinheit 99,3 %.

**Vergleichsbeispiel**

163,2 g IPN-azin, 250 ml Methanol, 350 ml Dioxan, 17,5 g Raney-Nickel und 4,2 g $NiCl_2 \cdot 6H_2O$ werden im 2 l-Rührautoklaven bei 110 °C und 100 bar $H_2$ bis zum Ende der $H_2$-Aufnahme gerührt. Nach Aufarbeitung erhält man 80,7 g IPDA = 47,4 % d. Th.

**Beispiel 18**

163,2 g (0,50 Mol) IPN-azin werden in einem 2 l-Autoklaven mit Begasungsrührer in 850 ml Methanol und 150 ml wasserfreiem flüssigen Ammoniak aufgelöst. Nach Zugabe von 50 g 5 % Ru/C und 70 ml Wasser wird unter Rühren Wasserstoff bis 10 MPa aufgepreßt und auf 110 °C erhitzt. Nach beendeter Hydrierung wird abgekühlt und entspannt; dann werden der Katalysator abfiltriert, der Ammoniak und das Lösungsmittel abgezogen und der Rückstand im Vakuum über eine Kolonne destilliert.

Hauptlauf: 144,6 g (85 % d. Th.) IPDA
$Kp_{0,3}$: 74 - 77 °C
Rückstand: 8,0 g

Der abfiltrierte Katalysator wurde ohne Ergänzung von Filtrationsverlusten erneut in 70 ml Wasser aufgeschlämmt und für weitere Versuche derselben Ansatzgröße jeweils eingesetzt.

| Nummer des erneuten Ansatzes | IPDA-Ausbeute |
|---|---|
| 2. | 86,1 % d. Th. |
| 3. | 86,0 % d. Th. |
| 4. | 87,5 % d. Th. |
| 5. | 86,0 % d. Th. |
| 6. | 89,0 % d. Th. |
| 7. | 88,3 % d. Th. |
| 8. | 86,7 % d. Th. |

Die Produktreinheiten lagen jeweils bei 98 - 99 % (GC-Bestimmung). Die Katalysatorzyklen ließen sich ohne Ausbeuteeinbuße noch weiter fortsetzen.

## Patentansprüche

1. Bis(3-cyano-3,5,5-trimethyl-cyclohexyliden)-azin (=IPN-azin)

2. Verfahren zur Herstellung des IPN-azins gemäß Anspruch 1, dadurch gekennzeichnet, daß man 1,3,3-Trimethyl-5-oxo-cyclohexan-carbonitril (=IPN) und einer Quelle für Hydrazin im Molverhältnis von im wesentlichen 2 zu 1 in Gegenwart eines Lösungsmittels umsetzt und, soweit erwünscht, das IPN-azin mittels üblicher Methoden aus dem Reaktionsgemisch isoliert.

3. Verfahren nach Anspruch 2, dadurch gekennzeichnet, daß man als Quelle für Hydrazin Hydrazinhydrat oder wäßrige Lösungen derselben und als Lösungsmittel ein organisches Lösungsmittel verwendet.

4. Verfahren nach Anspruch 2 oder 3, dadurch gekennzeichnet, daß man als Lösungsmittel einen niederen Alkohol oder niederen Ether verwendet.

5. Verfahren zur Weiterverarbeitung des IPN-azins gemäß Anspruch 1 zu 3-(Aminomethyl)-3,5,5-trimethylcyclohexylamin (IPDA), dadurch gekennzeichnet, daß man das IPN-azin in Gegenwart eines organischen Lösungsmittels, Ammoniak und eines Katalysatorsystems aus der Reihe (a) eines kobalt- oder nickelhaltigen Raney-Katalysators und eines Cokatalysators aus der Reihe von Salzen der Elemente Aluminium, Kobalt, Nickel, Lanthan, Yttrium, Cer, Ru, Rh, Pd, Ir und Pt oder von trägergebundenen Edelmetallen aus der Reihe Ru, Rh, Pd, Ir, Pt oder (b) eines trägergebundenen Ruthenium-, Palladium-oder Platinkatalysators bei einem Druck von 3 bis 30 MPa und einer Temperatur von 50 bis 150 °C mit Wasserstoff hydriert und das Reaktionsgemisch nach Abtrennung der Feststoffe destillativ aufarbeitet.

6. Verfahren nach Anspruch 5, dadurch gekennzeichnet, daß man die Hydrierung bei einem Druck von 3 bis 15 MPa und einer Temperatur von 80 bis 120 °C durchführt.

7. Verfahren nach Anspruch 5 oder 6, dadurch gekennzeichnet, daß man pro kg IPN-azin 50 bis 500 g Ammoniak einsetzt.

8. Verfahren nach einem oder mehreren der Ansprüche 5 bis 7,

dadurch gekennzeichnet,
daß man als Katalysator Raney-Nickel oder Raney-Kobalt und als Cokatalysator mindestens ein Chlorid der Elemente Al, Co, Ni, Y, La und Ce einsetzt.

**9.** Verfahren nach einem oder mehreren der Ansprüche 5 bis 8,
dadurch gekennzeichnet,
daß man als Lösungsmittel einen $C_1$- bis $C_4$-Alkohol, einen Ether mit bis zu 6 C-Atomen oder einen aliphatischen oder cycloaliphatischen Kohlenwasserstoff einsetzt.

**10.** Verfahren nach einem oder mehreren der Ansprüche 5 bis 9,
dadurch gekennzeichnet,
daß man das IPN-azin in Form einer Suspension, erhalten nach dem Verfahren gemäß einem der Ansprüche 2 bis 4, einsetzt.

**11.** Verfahren nach einem oder mehreren der Ansprüche 5, 6, 7, 9 und 10,
dadurch gekennzeichnet,
daß man als Katalysatorsystem (b) einen trägergebundenen Rutheniumkatalysator verwendet, wobei als Trägermaterial vorzugsweise Kohle oder Aluminiumoxid in feinverteiler Form vorliegt.

## Claims

**1.** Bis(3-cyano-3,5,5-trimethylcyclohexyliden)azine (= IPN azine)

**2.** A process for the production of the IPN azine according to Claim 1, characterized in that 1,3,3-trimethyl-5-oxocyclohexanecarbonitrile (= IPN) and a source of hydrazine in the molar ratio of essentially 2 to 1 are reacted in the presence of a solvent and, if desired, the IPN azine is isolated from the reaction mixture by means of usual methods.

**3.** A process according to Claim 2, characterized by the use of hydrazine hydrate or aqueous solutions thereof as a source of hydrazine and the use of an organic solvent as solvent.

**4.** A process according to Claim 2 or 3, characterized in that a lower alcohol or lower ether is used as solvent.

**5.** A process for the subsequent processing of the IPN azine according to Claim 1 to 3-(aminomethyl)-3,5,5-trimethyl-cyclohexylamine (IPDA), characterized in that the IPN azine is hydrogenated with hydrogen in the presence of an organic solvent, ammonia and a catalyst system from the series (a) of a cobalt- or nickel-containing Raney catalyst and a co-catalyst from the series of salts of the elements aluminium, cobalt, nickel, lanthanum, yttrium, cerium, ruthenium, rhodium, palladium, iridium and platinum or of supported noble metals from the series ruthenium, rhodium, palladium, iridium and platinum or (b) of a supported ruthenium, palladium or platinum catalyst at a pressure of 3 to 30 MPa and a temperature of 50 to 150 °C and the reaction mixture, after removal of the solids, worked up by distillation.

**6.** A process according to Claim 5, characterized in that the hydrogenation is carried out at a pressure of 3 to 15 MPa and a temperature of 80 to 120 °C.

**7.** A process according to Claim 5 or 6, characterized in that 50 to 500 g ammonia are used per kg IPN azine.

**8.** A process according to one or more of Claims 5 to 7, characterized in that Raney nickel or Raney cobalt is used as catalyst and at least one chloride of the elements aluminium, cobalt, nickel, yttrium, lanthanum and cerium is used as cocatalyst.

**9.** A process according to one or more of Claims 5 to 8, characterized in that a $C_1$ to $C_4$ alcohol, an ether with up to 6 C atoms or an aliphatic or cycloaliphatic hydrocarbon is used as solvent.

**10.** A process according to one or more of Claims 5 to 9, characterized in that the IPN azine is used in the form of a suspension obtained by the process according to one of Claims 2 to 4.

**11.** A process according to one or more of Claims 5, 6, 7, 9 and 10, characterized in that a supported ruthenium catalyst is used as catalyst system (b), carbon or aluminium oxide in finely divided form preferably being

present as the support material.

## Revendications

1. Bis(3-cyano-3,5,5-triméthyl-cyclohexylidène)-azine (= IPN-Azide).

2. Procédé de préparation de l'IPN-azine selon la revendication 1, caractérisé en ce qu'on fait réagir le 1,3,3-triméthyl-5-oxo-cyclohexane carbonitrile (=IPN) et une source d'hydragine en proportion molaire de 2 à 1 essentiellement en présence d'un solvant et, dans la mesure où on le souhaite, on isole du milieu réactionnel l'IPN-azine au moyen des procédés usuels.

3. Procédé selon la revendication 2, caractérisé en ce qu'on utilise comme source d'hydrazine de l'hydrate d'hydrazine ou ses solutions aqueuses et comme solvant, un solvant organique.

4. Procédé selon la revendication 2 ou 3, caractérisé en ce qu'on utilise comme solvant un alcool inférieur ou un éther inférieur.

5. Procédé de transformation de l'INP-azine selon la revendication 1, en 3-(aminométhyl)-3,5,5-triméthylcyclohexylamine(IPDA), caractérisé en ce qu'on hydrogène avec de l'hydrogène l'IPN-azine en présence d'un solvant organique, de l'ammoniac et d'un système catalytique de la série (a) d'un catalyseur Raney contenant du nickel ou du cobalt et d'un cocatalyseur de la série des sels des éléments aluminium, cobalt, nickel, lanthane, yttrium, cérium, Ru, Rh, Pd, Ir et Pt ou de métaux précieux liés à un support de la série Ru, Rh, Pd, Ir, Pt ou (b) d'un catalyseur de platine, palladium, ruthénium lié à un support, sous une pression de 3 à 30 MPa et une température de 50 à 150°C, et après séparation des solides, on traite par distillation le mélange réactionnel.

6. Procédé selon la revendication 5,caractérisé en ce qu'on réalise l'hydrogénation sous une pression de 3 à 15 MPa et une température de 80 à 120°C.

7. Procédé selon la revendication 5 ou 6, caractérisé en ce qu'on utilise par kg d'IPN-azine de 50 à 500 g d'ammoniac.

8. Procédé selon une ou plusieurs des revendications 5 à 7, catactérisé en ce qu'on utilise comme catalyseur du nickel Raney ou du cobalt Raney et comme cocatalyseur au moins un chlorure des éléments Al, Co, Ni, Y, La et CE.

9. Procédé selon une ou plusieurs des revendications 5 à 8, caractérisé en ce qu'on utilise comme solvant un alcool $C_1$ à $C_4$, un éther avec jusqu'à 6 atomes de C ou un hydrocarbure aliphatique ou cycloalyphatique.

10. Procédé selon une ou plusieurs des revendications 5 à 9, caractrérisé en ce qu'on utilise l'IPN-azine sous forme d'une suspension obtenue selon le procédé suivant l'une des revendications 2 à 4.

11. Procédé selon une ou plusieurs des revendications 5, 6, 7, 9 et 10, caractérisé en ce qu'on utilise comme système catalytique (b) un catalyseur de ruthénium lié à un support, dans lequel le matériau support est de préférence du charbon ou de l'oxyde d'aluminium sous forme finement divisée.

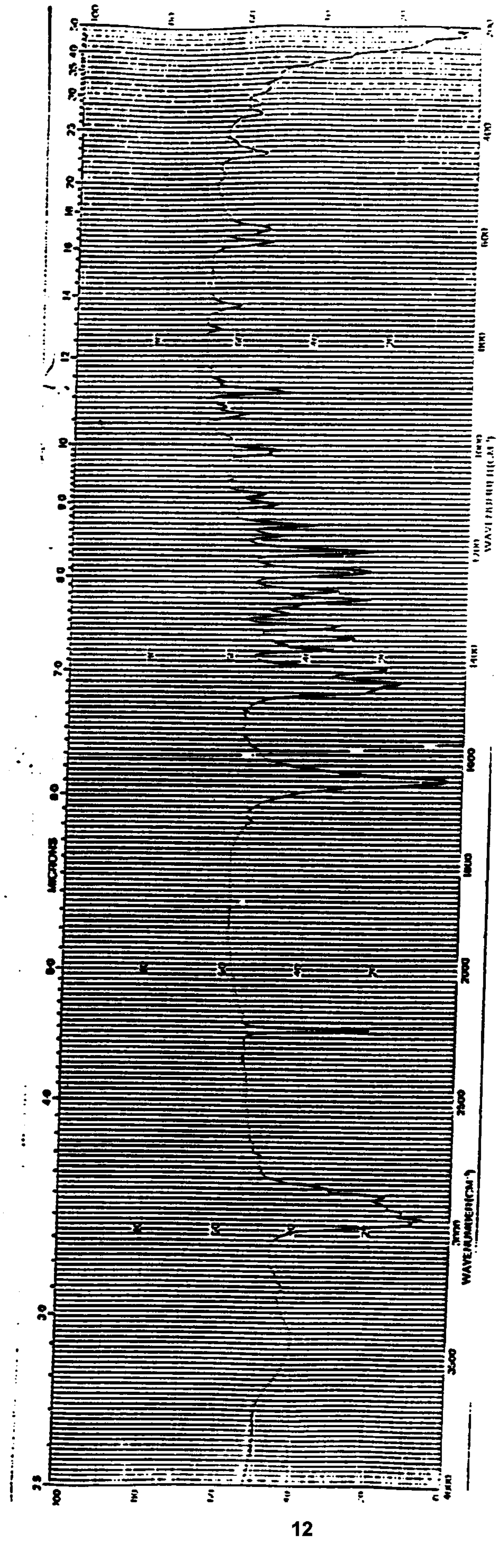


Figur 1/1